# EUROPEAN PATENT SPECIFICATION

(11) **EP 1 073 431 B1**
(45) Date of publication and mention of the grant of the patent: **07.07.2004**
(21) Application number: 99947024.8
(22) Date of filing: 28.04.1999
(51) Int. Cl.: A61K 31/165, A61K 31/13, A61K 31/41, A61K 31/44, A61K 31/495, A61P 1/00

(54) **USE OF NMDA ANTAGONISTS FOR TREATMENT OF IRRITABLE BOWEL SYNDROME**
VERWENDUNG VON NMDA ANTAGONISTEN ZUR BEHANDLUNG DES REIZKOLONS
UTILISATION D'ANTAGONISTES DE NMDA POUR LE TRAITEMENT DU SYNDROME DU COLON IRRITABLE

(30) Priority: 28.04.1998 SE 9801494; 18.11.1998 SE 9803954
(43) Date of publication of application: 07.02.2001
(73) Proprietor: AstraZeneca AB, 151 85 Södertälje (SE)
(72) Inventor: ASGHAR, Aziz, School of Biomedical Sciences, Leeds LS2 9NQ (GB); CABERO, José, Luis, S-431 83 Mölndal (SE); DRAY, Andrew, AstraZeneca R&D Montreal, St. Laurent, Quebec H4S 1Z9 (CA); KING, Anne, School of Biomedical Services, Leeds, LS2 9NQ (GB)
(86) International application number: PCT/SE1999/000702
(87) International publication number: WO 1999/055323

(56) References cited:
- WO-A1-97/14415
- WO-A2-97/09317

## Description

### Field of the invention

The present invention relates to the use of compounds having NMDA antagonist activity for treating certain conditions in the gastro intestinal tracts, such as functional gastrointestinal disorders, and in particular irritable bowel syndrome (IBS), where the condition known as visceral hypersensitivity may be a major contributory factor in the observed symptoms. The invention also relates to pharmaceutical compositions intended for the treatment of IBS.

### Background of the invention

Compounds having NMDA (N-methyl-D-aspartate) antagonist activity are known in the art, for example see Watkins et al., Trends in Pharmacological Science, 11:25, 1990.

In particular certain compounds are disclosed in EP-A 279937 as having NMDA antagonist activity and are useful for treating various CNS disorders such as epilepsy and Parkinson's disease. In particular the compound known as remacemide is known from EP-A 279937 as an NMDA antagonist and has also been shown to act as a sodium channel antagonist (Wamil et al., Epilepsy Research 23:1. 1996). It has now surprisingly been found that antagonists of the NMDA receptor have an attenuating effect on the visceromotor response to colorectal distention in rats when dosed intravenously but not when dosed intrathecaly. This observation coupled with the observation that the compounds also show an attenuating effect in a model of pelvic nerve afferent activity, would suggest that the effect of these NMDA antagonists is dependant at least in part on a peripheral component. It docs not however, rule out an additional action at the spinal or supra-spinal level, in the attenuation of the response to colorectal distension. As a result it is expected that compounds having NMDA antagonist activity which in some cases may be combined with sodium channel antagonist activity will be useful for the treatment of certain conditions in the gastro intestinal tracts where the phenomenon of visceral hypersensitivity may be involved, such as functional bowel disorders, and in particular irritable bowel syndrome.

Suitable NMDA antagonists include those listed in WO 94/13295 such as a) channel blockers, i.e. antagonists which operate in an uncompetitive or non-competitive manner to block the NMDA receptor channel, b) receptor antagonists that compete with NMDA to act at the NMDA binding site, c) agents acting at either the glycine co-agonist site of may of the several modulation sites such as the zinc site, the magnesium site, the redox modulatory site, or the polyamine site, d) agents which inhibit the downstream effects of NMDA receptor stimulation such as agents which inhibit the activation of protein kinase C activation by NMDA stimulation, antioxidants, and agents that decrease phosphatidylinositol metabolism.

The hypersensitive state, such as that which may occur in patients with functional bowel disorders, may occur as a result of excessive receptor activation. Hence, antagonists which operate in an uncompetitive or non-competitive manner, may offer an advantage as they can only block the receptor when it is in its activated state and not when it is in its non-activated form. Thus excess receptor activity will be curtailed.

Examples of preferred compounds useful for the invention include but are not limited to memantine (Merz) and remacemide and their metabolites

Particularly suitable compounds are those disclosed in EPA 279937, such as a compound of formula (I): 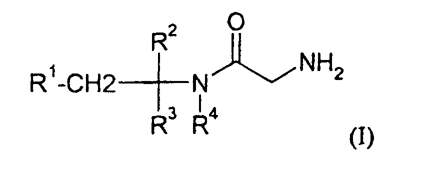 where:
R¹ and R² are independently phenyl or 4-fluorophenyl;
R³ is hydrogen, C₁₋₆alkyl or methoxycarbonyl;
R⁴ is hydrogen or methyl;
and metabolites and isomers thereof both as free base and pharmaceutically acceptable salts thereof.

Preferred compounds of formula (I) include 2-amino-N-(1-methyl-1,2-diphenylethyl)acetamide (remacemide) or a metabolite thereof, such as the compound 2,3-diphenyl-2-propylamine or a pharmaceutically acceptable salt thereof which has the following structure:

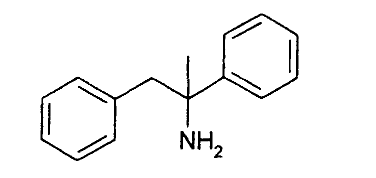

Other preferred compounds include those disclosed in WO 93/20052, in particular (S)-1-phenyl-2-(2-pyridyl)ethanamine as well as the compounds mentioned in the experimental section herein. Certain compounds mentioned herein are capable of existing in stereoisomeric forms including enantiomers and the invention extends to each of these individual stereoisomeric forms and to any mixtures thereof including racemates. The invention also extends to any tautomeric forms of the compounds mentioned and mixtures thereof.

Suitable salts of the above noted compounds include all known pharmaceutically acceptable salts such as acid addition salts and preferably hydrochloride salts.

Compounds which possess anti-inflammatory properties are useful in the prevention of clinical hyperalgesia and other pathologies associated with chronic pain such as neuropathies and joint inflammation. Particular inflammatory disorders which can be treated include arthritic conditions, eczema, psoriasis, dermatitis and other inflammatory conditions such as sunburn; inflammatory eye conditions such as uveitis and conjunctivitis; lung disorders in which inflammation is involved such as asthma and bronchitis; conditions of the GI tract including aphthous ulcers, gingivitis, Crohn's disease, atrophic gastritis, gastritis varialoforme, ulcerative colitis, coeliac disease, regional iletis, peptic ulceration, IBS, pyresis, pain, including inflammatory induced pain, and other damage to the GI tract, for example damage from infections by, for example, Helicobacter pylori, or undesirable side effects from treatments with non-steroidal anti-inflammatory drugs.

### Outline of the invention

In a preferred embodiment it has been found that certain NMDA antagonists arc expected to be useful for the treatment of certain conditions in the GI tract, in particular functional bowel disorders.

In a further aspect the invention therefore provides use of an NMDA antagonist for the treatment or prevention of irritable bowel syndrome (IBS). Suitable NMDA antagonists include those listed above. In particular a preferred aspect of the invention relates to the use of non-competitive NMDA antagonists such as memantine for the treatment of IBS. Other preferred compounds for the treatment or prevention of IBS include remacemide, and also compounds of formula I, such as (S)-1-phenyl-2-(2-pyridyl)ethanamine, 2-amino-N-(1,2-diphenylethyl)acetamide hydrochloride, alpha-phenyl-1H-pyrazole-1-ethanamine hydrochloride, (+)-N-ethyl-1-phenyl-2-(3-pyrazine)ethanamine hydrochloride and 2-amidino-6-(2-amino-2-phenyl)-ethylpyridine trihydrochloride.

In a preferred embodiment the invention provides the use of a compound having NMDA antagonist activity or a pharmaceutically acceptable salt thereof, for the preparation of a medicament for the treatment of IBS.

The invention also provides the use of a compound having NMDA antagonist activity in the manufacture of a medicament for use in the prevention or treatment of IBS, and a pharmaceutical composition comprising such a compound and a pharmaceutical acceptable carrier.

Other diseases which may be treated with the compounds of the invention include functional gastrointestinal disorders as defined by the Rome group in *"The Functional Gastrointestinal Disorders",* D. Drossman ed., Little Brown & Co., 1994, p.p. 370. In particular: irritable bowel syndrome and functional dyspepsia (non-ulcer dyspepsia) but also functional chest pain of presumed oesophageal origin, functional heartburn, functional dysphagia, non-cardiac chest pain, symptomatic gastro-oesophageal disease, gastritis, aerophagia, functional constipation, functional diarrhea, burbulence, chronic functional abdominal pain, functional biliary pain, functional incontinence, functional ano-rectal pain, pelvic floor dyssenergia, un-specified functional ano-rectal disorder. Additional conditions include cholecystalgia, interstitial cystitis, dysmenorrhea, dyspareunia, cancer related pain, migraine, osteoarthritis and rheumatoid arthritis.

### Use of the invention

Suitable daily dose ranges of the compound having NMDA antagonist activity arc from about 1.0 mg/kg to about 100 mg/kg. Unit doses may be administered conventionally once or more than once a day, for example, 2, 3, or 4 times a day, more usually I or 2 times a day.

The following examples illustrate the invention.

### Example 1

### Effects of non-competitive NMDA glutamate receptor antagonists on the visceromotor response (VMR) elicited by colorectal distension (CRD)

### Methods:

### Animals

Adult male Sprague-Dawley rats (250-350g, Harlan, San Diego, CA) served as animal subjects. Rats were housed 5-6 per cage, allowed free access to food and water, and were maintained on a 12 h light-dark cycle (lights on between 06.00 and 18.00 h).

### Surgical preparation

Rats were deeply anesthetized with pentobarbital sodium (45 mg/kg, Nembutal, Abbott Labs, North Chicago, IL) administered intraperitoneally. Electrodes (Teflon coated stainless steel wire, Cooner Wire Sales, Chatworth, CA) were stitched into the external oblique musculature, just superior to the inguinal ligament, for electromyographic (EMG) recording. The electrode leads were tunneled subcutaneously and exteriorized at the nape of the neck for future access. Some animals were also implanted with venous catheters in the femoral vein to enable i.v. administration of drugs. For intrathecal (i.t.) drug administration, an i.t. catheter (PE-10 tubing, 8.5 cm long) was inserted through the dura overlying the atlanto-occipital junction and threaded to the level of the lumbar enlargement (Yaksh and Rudy, 1976). The venous or i.t. catheter was surgically anchored to musculature at the back of the neck, and externalized with the electrode leads. The wounds were closed in layers with 4-0 silk. Rats were housed singly and allowed to recuperate for at least 3-5 days prior to testing.

### Behavioral testing

The stimulus employed has been previously described (Gebhart and Sengupta, 1996). Briefly, the descending colon and rectum were distended by pressure-controlled air inflation of a 6 cm long flexible latex balloon tied around a flexible tube (Tygon). The balloon was lubricated (Surgilube, E. Fougera and Co., Melville, NY), inserted intra-anally and anchored by taping the balloon catheter to the base of the tail. Noxious phasic CRD (80 mmHg, 20 s) was achieved with the aid of a device (developed in house at Astra Hässle.) Intracolonic pressure was continuously monitored on line. The behavioral response quantified was the visceromotor response, a contraction of the abdominal and hindlimb (Ness and Gebhart, 1988). EMG activity in the external oblique musculature was quantified by computing the average amplitude (Dr. Alfred Bayati Astra Hässle). Each distension trial lasted 60 s and EMG activity was quantified 20 s before distension (baseline), during distension, and 20 s after distension. The increase in EMG amplitude during distension over baseline was recorded as the response.

### Experimental protocol

On the day of testing, animals were briefly anesthetized with Metophane®, and the balloon was inserted and secured in place as described above. Rats were allowed to recover for 30-40 min, following which two stable control responses to CRD (80 mmHg, 20 s, 4 min interstimulus interval) were obtained.

Drugs were administered i.v. into the femoral vein through the indwelling catheter. All doses were administered in a volume of up to 230 µl followed by a flush with 100 µl of preservative-free saline over a period of 30s. Dose response curves were generated using a cumulative dosing paradigm. The first i.v. injection was made 2 min after the second control response. Subsequent doses were injected 8 min apart, thus allowing two distensions after each dose. Data are reported as the average response to the two distensions.

In one group of animals, memantine was administered to the lumbar enlargement through the indwelling i.t. catheter with the aid of a 16 gauge injection needle connected to a 25 ul Hamilton syringe by a length of polyethylene tubing (PE-10). All doses were administered in a volume of 5 µl followed by a flush with 10 µl of preservative-free saline over a period of 1 min. The progress of the injection was continuously monitored by following the movement of an air bubble in the tubing. The dose response curve was generated using a cumulative dosing paradigm. The first i.t. injection was made 2 min after the second control response. Subsequent doses were injected 8 min apart, thus allowing two distensions after each dose. Data are reported as the average response to the two distensions.

### Drugs

Drugs used in the present study were memantine hydrochloride (Research Biochemicals International, Natick, MA), and 2-amino-N-(1,2-diphenylethyl)acetamide hydrochloride, alpha-phenyl-1H-pyrazole-1-ethanamine hydrochloride, (+)-N-ethyl-1-plenyl-2-(3-pyrazine)ethanamine hydrochloride and 2-amidino-6-(2-amino-2-phenyl)-ethylpyridine trihydrochloride (Astra Arcus, Rochester, NY). Stock solutions were freshly prepared by dissolving the drugs in distilled water, and then diluted as needed.

### Results:

All drugs administered i.v. produced a dose-dependent attenuation of the VMR to noxious CRD (80 mmHg) in naive animals without producing any apparent motor effects. At the most effective dose tested, memantine (10 mg/kg) attenuated the VMR to 28 % of control, 2-amino-N-(1,2-diplienylethyl)acetamide hydrochloride (60 mg/kg) to 40 % of control, alpha-phenyl-1H-pyrazole-1-ethanamine hydrochloride (100 mg/kg) to 10% of control, (+)-N-ethyl-1-phenyl-2-(3-pyrazine)ethanamine hydrochloride (60 mg/kg) to 5 % of control and 2-amidino-6-(2-amino-2-phenyl)-ethylpyridine trihydrochloride to 13 % of control.

In contrast, memantine administered i.t. (1-100 nmol) was without effect in diminishing the VMR to CRD. This is supported by other studies wherein NMDA receptor antagonists administered i.t. were without effect on normal visceral nociceptive reflexes, except in doses that produce motor impairment (Rice and McMahon, 1994; Coutinho et al., 1996a; Ide et al., 1997).

These data suggest that memantine as well as the other open channel blockers tested may be interacting with peripheral NMDA receptors.

Therefore it appears that activity at peripheral NMDA receptors plays a role in mediating responses to CRD.

Results are presented in Figures 1 to 4, which figures show the following:
Fig 1. Illustrates the effect of intravenous (i.v.) administration of memantine hydrochloride on viscero-motor responses (VMR) to noxious colorectal distension of concious rats. Memantine dose-dependantly attenuate VMR when given i.v. from 1-10 mg/kg.
Fig 2. Effect of intrathecal (i.t.) administration of memantine hydrochloride on viscero-motor responses (VMR) to noxious colorectal distension of concious rats. Memantine did not attenuate VMR when given i.t. up to a dose of 100 nmol.
Fig 3. Effect of intravenous (i.v.) administration of four compounds on viscero-motor responses (VMR) to noxious colorectal distension of concious rats. All four compounds dose-dependently (1-10 mg/kg) attenuated VMR.
   Compound 1 is 2-amidino-6-(2-amino-2-phenyl)-ethylpyridine trihydrochloride
   Compound 2 is 2-amino-N-(1,2-diphenylethyl)acetamide hydrochloride
   Compound 3 is (+) N-ethyl-1-phenyl-2-(3-pyrazine)ethanamine hydrochloride
   Compound 4 is alpha-phenyl-1H-pyrazote-1-ethanamine hydrochloride
Fig 4. Effect of intrathecal (i.t.) administration of three compounds on viscero-motor responses (VMR) to noxious colorectal distension of concious rats. None of the three compounds attenuated VMR up to a dose of 300nmol.
   Compound 1 is 2-amidino-6-(2-amino-2-phenyl)-ethylpyridine trihydrochloride
   Compound 2 is 2-amino-N-(1,2-diphenylethyl)acetamide hydrochloride
   Compound 4 is alpha-phenyl-1H-pyrazote-1-ethanamine hydrochloride

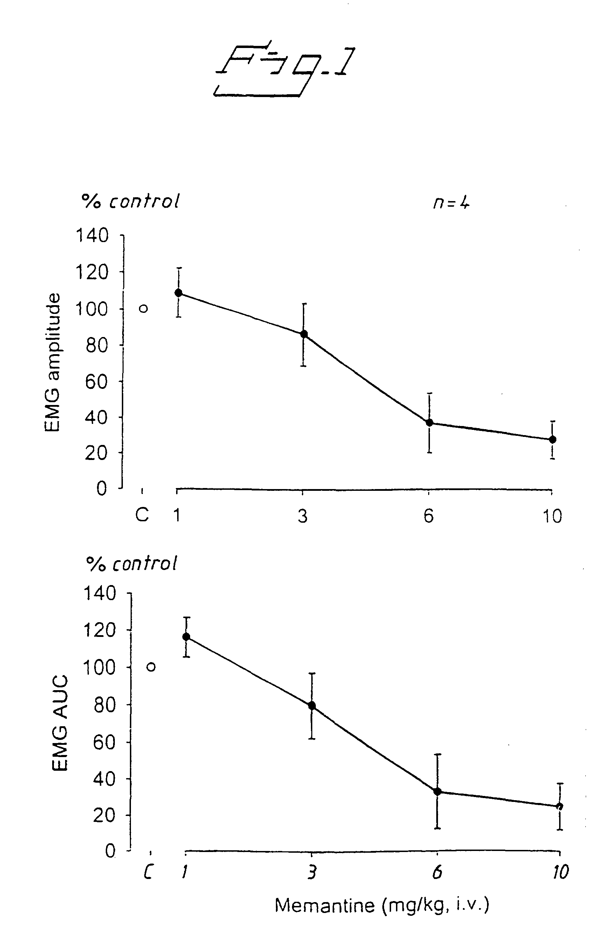

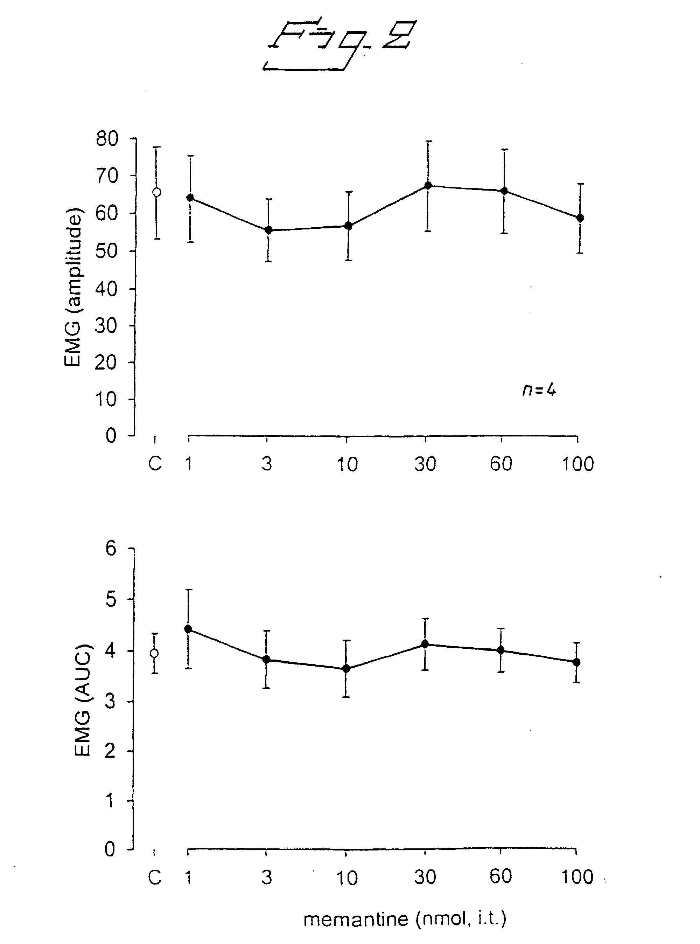

### Example 2

### Effects of non-competitive NMDA glutamate receptor antagonists on pelvic afferent nerve activity

*General procedures:* Male Sprague-Dawley rats 425-450 g) were anesthetized initially with sodium pentobarbital (40-45 mg/kg ip) and maintained with α-chloralose (60 mg kg⁻¹ h⁻¹). The trachea was cannulated for mechanical ventilation with room air. The left common carotid artery was cannulated for recording blood pressure. The femoral artery and vein were catheterized for injection of drug and anesthetic, respectively. Rats were paralyzed with pancuronium bromide (0.3 mg/kg i.v.) and ventilated with room air (55-60 strokes/min and 3-4 ml stroke volume). Supplemental doses of pancuronium bromide (0.2-0.3 mg kg⁻¹ h⁻¹) were given to maintain paralysis during the course of experiment. The mean arterial blood pressure was monitored continuously and maintained at >80 mm Hg with supplemental intravenous injection of 5% dextrose in saline given in a bolus of 1 to 1.5 ml as required. The core body temperature was maintained at 36°C by a hot-water-circulating heating pad underneath the rat and a feedback-controlled heat lamp (thermoprobe inserted into the thoracic esophagus). At the end of an experiment, the rat was killed by an overdose of intravenous pentobarbital sodium.

*Surgical procedure:* The lower abdomen was exposed by a 3-4 cm long incision laterally at the left flank. The urinary bladder was emptied and catheterized (PE-100) through the fundus. The urethra was ligated close to its entry to the penis and urine was constantly evacuated via the fundic catheter.

The pelvic nerve was approached near the major pelvic ganglion and isolated. A pair of Teflon-coated stainless steel wires stripped at the tips were wrapped around the pelvic nerve and sealed with non-reactive Wacker gel. The hypogastric, pudendal, and femoral nerves were isolated and transected. The sciatic nerve was approached through the ischiatic notch and transected. The abdomen was closed with silk sutures.

The lumbosacral spinal cord was exposed by laminectomy (T₁₃-S₂) and the rat was suspended from thoracic vertebral and ischial spinal clamps. The dorsal skin was reflected laterally and tied to make a pool for mineral oil. The dura was carefully removed and the spinal cord was covered with warm (37°C) mineral oil. For colorectal distension (CRD), a 6 - 7-cm long, 2 - 3 cm diameter flaccid, flexible latex balloon was inserted into the descending colon and rectum as described above.

*Recordings of afferent nerve action potentials:* The S₁ dorsal root was identified and decentralized at its entry to the spinal cord. Recordings were made from the distal cut end of the central processes of primary afferent fibers. a length of nerve fiber was draped over a black micro-base plate immersed in warm (37°C) mineral oil. The dorsal rootlet was split into thin bundles and a fine filament was isolated from the bundle to obtain a single unit. Electrical activity of single units was recorded monopolarly by placing a teased fiber over one arm of a bipolar silver-silver chloride electrode; a fine strand of connective tissue was placed across the other pole of the electrode. Action potentials were monitored continuously by analog delay and displayed on a storage oscilloscope after initial amplification through a low-noise ac differential amplifier. Action potenlials were processed through a window discriminator and the frequency of impulses were counted (is binwidth) on-line using the spike2/ced 1401 data acquisition program. Peri-stimulus time histograms (PSTH), urinary bladder or colonic distending pressures, and blood pressure were displayed on-line.

*Experimental protocol:* Pelvic nerve input to the s, dorsal root was identified first by electrical stimulation of the pelvic nerve (a single 0.5 ms square-wave pulse at 5-8 mA). The organ innervated was identified by response to phasic CRD (80mm Hg, 2-3s). If a fiber responded to CRD, a stimulus-response function to phasic distending pressures of 5, 10, 20, 30, 40. 60, 80, and 100 mm Hg, 30s each at 4 min intervals was determined.

The effect of the NMDA-antagonist, memantine, was tested on responses of mechanosensitive pelvic nerve afferents to 80 mm Hg of CRD. The drug was administered intra-arterially in a cumulative dose paradigm. Each dose of the drug was given 2 min before CRD. A cumulative dose-response relationship for memantine was obtained by giving 1, 3, 6 and 10 mg/kg.

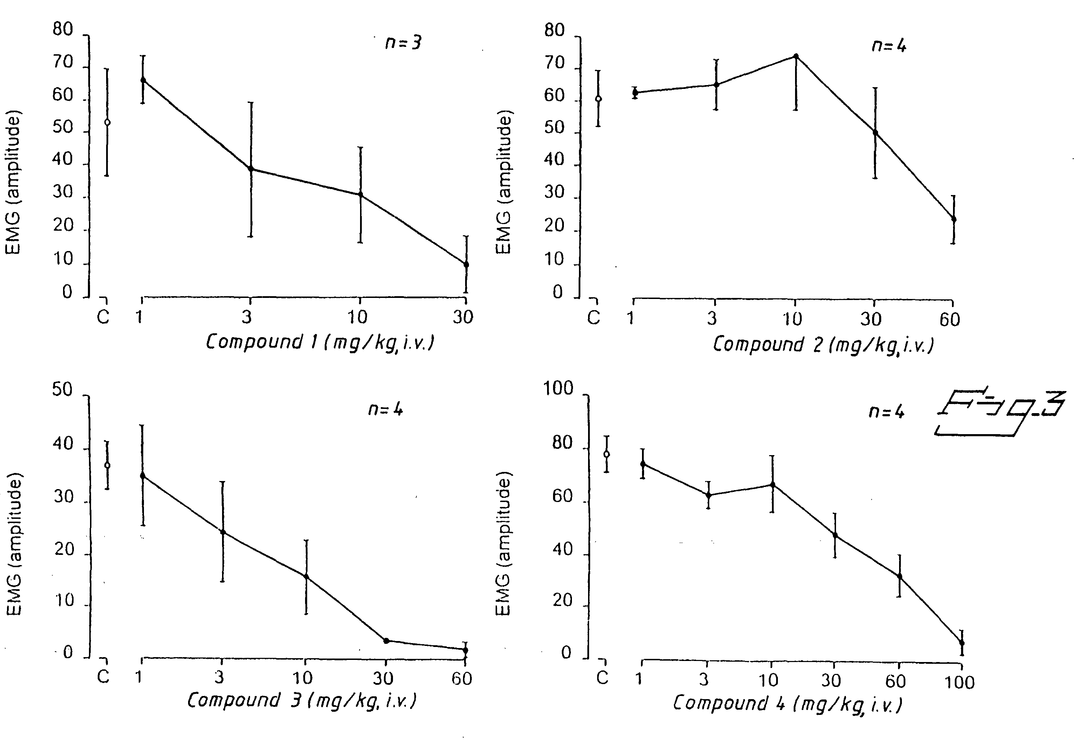

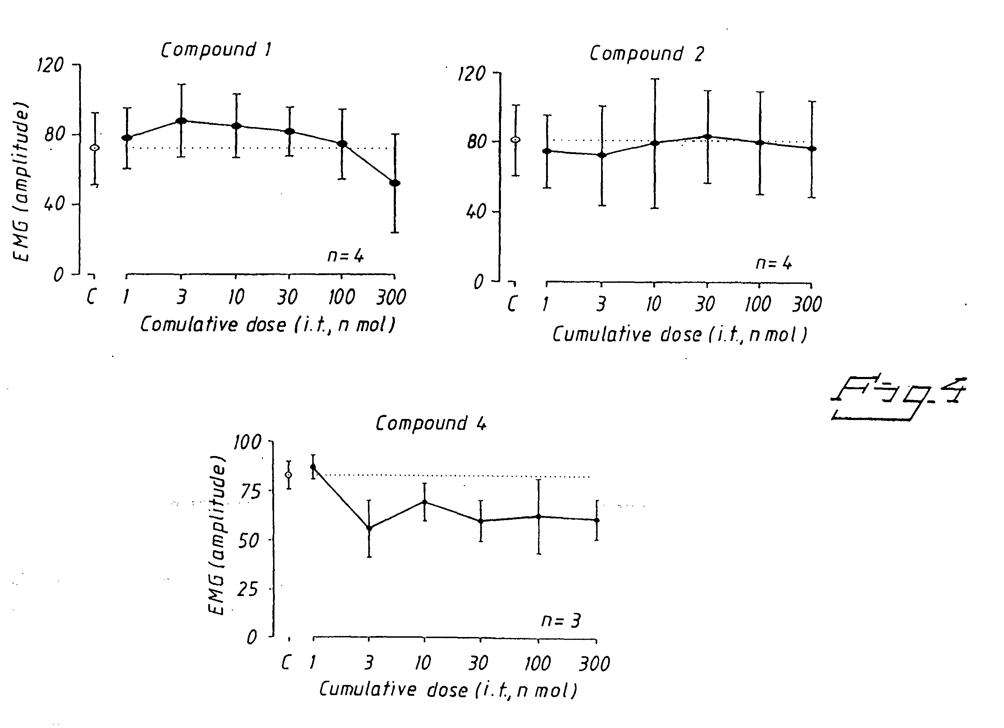

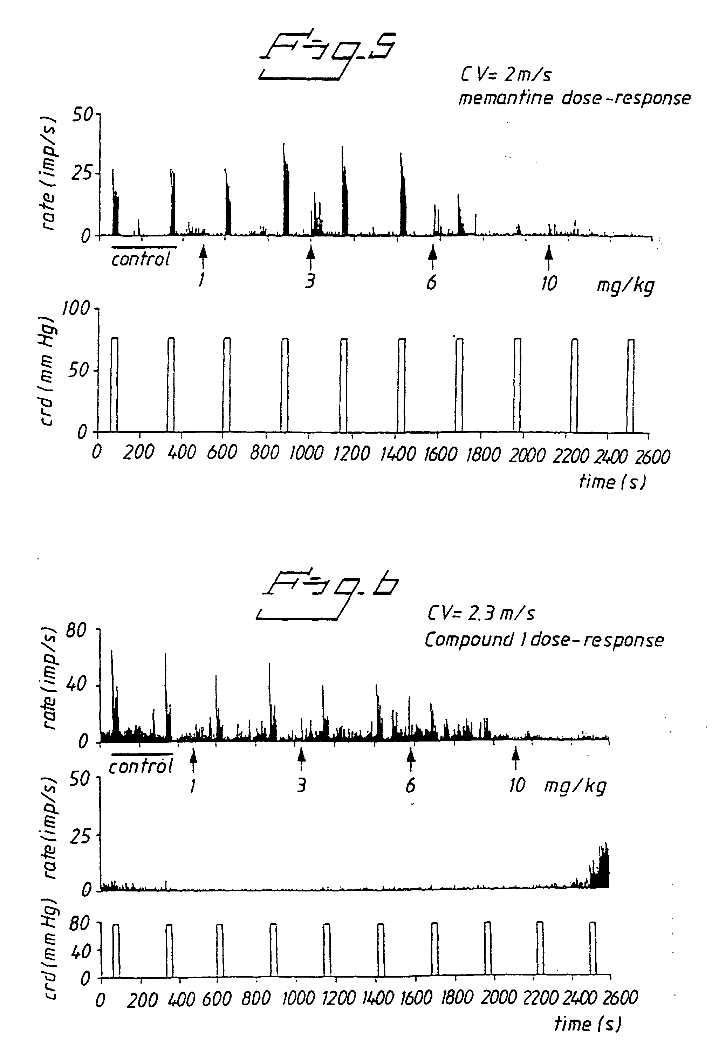

Figure 5 shows the results for memantine and Figure 6 shows the corresponding results with 2-amidino-6-(2-amino-2-phenyl)-ethylpyridine.

*Results and conclusion:* Intra-arterially injected memantine reduced, in a dose-dependent fashion, the pelvic nerve activity elicited by distention of the colon (80 mm Hg), as can be seen from Figure 5.

The observations hereby provided are consistant with a model in which the non-competitive NMDA-antagonist memantine reduces the pelvic nerve activity elicited by colorectal distention by a peripheral mechanism of action.

The data shown in Figure 6 was obtained when the experiment was repeated with 2-amidino-6-(2-amino-2-phenyl)-ethylpyridine.

### References:

Coutinho S.V.. Meller S.T. and Gebhart, G.F. (1996a) Intracolonic zymosan produces visceral hyperalgesia in the rat that is mediated by spinal NMDA and non-NMDA receptors. *Brain Res.* **736**, 7-15.

Coutinho, S.V.. Urban, M.O. and Gebhart, G.F.(1996b) NMDA and non-NMDA receptors in the RVM modulate responses to colorectal distension from the inflamed colon. In: Abst. 8^{th} World Cong. on Pain, pg.251, IASP Press, Seattle, WA.

Gebhart, G.F. and Sengupta, J.N. (1996) Evaluation of visceral pain. In *Handbook of Methods in Gastrointestinal Pharmacology* (ed. Gaginella, T.S.), pp. 359-374, CRC Press, Boca Raton.

Ide, Y., Maehara, Y., Tsukahara, S., Kitahata, L.M. and Collins, J.G. (1997) The effects of an intrathecal NMDA receptor antagonist (AP5) on the behavioral changes induced by colorectal inflammation with turpentine in rats. *Life Sci.* **60**, 1359-1363.

Ness T.J. and Gebhart, G.F. (1988) Colorectal distension as a noxious visceral stimulus: physiologic and pharmacologic characterization of pseudaffective reflexes in the rat. *Brain Res.* **450,** 153-169.

Rice, A.S.C. and McMahon, S.B. (1994) Pre-emptive intrathecal administration of an NMDA receptor antagonist (AP5) prevents hyper-reflexia in a model of persistent visceral pain. *Pain* **57**, 335-340.

Yaksh, T.L. and Rudy, T.A. (1976) Chronic catheterization of the spinal subarachnoid space. *Physiol. Behav.* **17**, 1031-1036.

## Claims

1. Use of a compound having NMDA receptor antagonist activity wherein the compound is selected from the group consisting of a compound of formula (I): 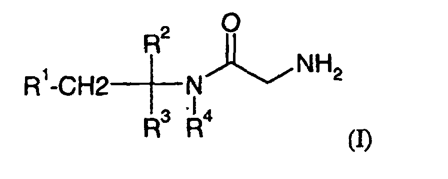 where:
R¹ and R² are independently phenyl or 4-fluorophenyl;
R³ is hydrogen, C₁₋₆ alkyl or methoxycarbonyl;
R⁴ is hydrogen or methyl; and metabolites and isomers thereof both as a free base and pharmaceutically acceptable salts thereof;
memantine, alpha-phenyl-1H-pyrazole-1-ethanamine, (+)-N-ethyl-1-phenyl-2-(3-pyrazine)ethanamine, 2-amidino-6-(2-amino-2-phenyl)-ethylpyridine trihydrochloride, 2,3-diphenyl-2-propylamine, (S)-1-phenyl-2-(2-pyridyl)ethanamine or a pharmaceutically acceptable salt thereof;
in the manufacture of a medicament for the treatment of irritable bowel syndrome (IBS).

2. Use according to claim 1 wherein the compound of formula (I) is remacemide or a pharmaceutically acceptable salt thereof.

3. Use according to claim 1 wherein the compound is 2,3-diphenyl-2-propylamine or a pharmaceutically acceptable salt thereof.

4. Use according to claim I wherein the compound is (S)-1-phenyl-2-(2-pyridyl)ethanamine or a pharmaceutically acceptable salt thereof.

5. Use according to claim I wherein the compound is memantine or a pharmaceutically acceptable salt thereof.

6. Use according to claim 1 where the compound is 2-amino-N-(1,2-diphenylethyl)acetamide, alpha-phenyl-1H-pyrazole-1-ethanamine, (+)-N-ethyl-1-phenyl-2-(3-pyrazine)ethanamine, or 2-amidino-6-(2-amino-2-phenyl)-ethylpyridine or a pharmaceutically acceptable salt thereof.

## Patentansprüche

1. Verwendung einer Verbindung mit NMDA-rezeptorantagonistischer Wirkung, wobei die Verbindung aus der aus einer Verbindung der Formel (I): 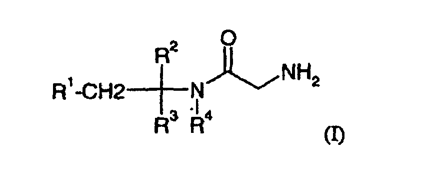 wobei:
R¹ und R² unabhängig voneinander für Phenyl oder 4-Fluorphenyl stehen;
R³ für Wasserstoff, C₁₋₆-Alkyl oder Methoxycarbonyl steht;
R⁴ für Wasserstoff oder Methyl steht; und Metaboliten und Isomeren davon, sowohl als freie Base als auch als pharmazeutisch unbedenkliche Salze davon;
Memantin, alpha-Phenyl-1H-pyrazol-1-ethanamin, (+)-N-Ethyl-1-phenyl-2-(3-pyrazin)ethanamin, 2-Amidino-6-(2-amino-2-phenyl)ethylpyridin-trihydrochlorid, 2,3-diphenyl-2-propylamin, (S)-1-Phenyl-2-(2-pyridyl)ethanamin und pharmazeutisch unbedenklichen Salzen davon bestehenden Gruppe ausgewählt ist;
bei der Herstellung eines Medikaments zur Behandlung von Reizkolon (Irritable Bowel Syndrome, IBS).

2. Verwendung nach Anspruch 1, wobei es sich bei der Verbindung der Formel (I) um Remacemid oder ein pharmazeutisch unbedenkliches Salz davon handelt.

3. Verwendung nach Anspruch 1, wobei es sich bei der Verbindung um 2,3-Diphenyl-2-propylamin oder ein pharmazeutisch unbedenkliches Salz davon handelt.

4. Verwendung nach Anspruch 1, wobei es sich bei der Verbindung um (S)-1-Phenyl-2-(2-pyridyl)ethanamin oder ein pharmazeutisch unbedenkliches Salz davon handelt.

5. Verwendung nach Anspruch 1, wobei es sich bei der Verbindung um Memantin oder ein pharmazeutisch unbedenkliches Salz davon handelt.

6. Verwendung nach Anspruch 1, wobei es sich bei der Verbindung um 2-Amino-N-(1,2-diphenylethyl)acetamid, alpha-Phenyl-1H-pyrazol-1-ethanamin, (+)-N-Ethyl-1-phenyl-2-(3-pyrazin)ethanamin oder 2-Amidino-6-(2-amino-2-phenyl)ethylpyridin oder ein pharmazeutisch unbedenkliches Salz davon handelt.

## Revendications

1. Utilisation d'un composé ayant une activité d'antagoniste des récepteurs de NMDA dans laquelle le composé est choisi dans le groupe constitué par un composé de formule (I) : 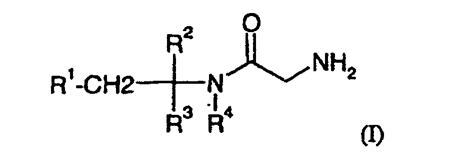 dans laquelle :
R¹ et R² sont indépendamment des groupes phényle ou 4-fluorophényle ;
R³ est un atome d'hydrogène ou un groupe alkyle en C₁₋₆ ou méthoxycarbonyle ;
R⁴ est un atome d'hydrogène ou un groupe méthyle ; et les métabolites et isomères de ce composé, tant sous forme de base libre que de ses sels pharmaceutiquement acceptables ;
la mémantine, l'alpha-phényl-1H-pyrazole-1-éthanamine, la (+)-N-éthyl-1-phényl-2-(3-pyrazine)-éthanamine, le trichlorhydrate de 2-amidino-6-(2-amino-2-phényl)éthylpyridine, la 2,3-diphényl-2-propylamine, la (S)-1-phényl-2-(2-pyridyl)éthanamine ou un de leurs sels pharmaceutiquement acceptables ;
dans la fabrication d'un médicament destiné au traitement du syndrome du côlon irritable (IBS).

2. Utilisation selon la revendication 1 dans laquelle le composé de formule (I) est le rémacémide ou un de ses sels pharmaceutiquement acceptables.

3. Utilisation selon la revendication 1 dans laquelle le composé est la 2,3-diphényl-2-propylamine ou un de ses sels pharmaceutiquement acceptables.

4. Utilisation selon la revendication 1 dans laquelle le composé est la (S)-1-phényl-2-(2-pyridyl)-éthanamine ou un de ses sels pharmaceutiquement acceptables.

5. Utilisation selon la revendication 1 dans laquelle le composé est la mémantine ou un de ses sels pharmaceutiquement acceptables.

6. Utilisation selon la revendication 1 dans laquelle le composé est le 2-amino-N-(1,2-diphényléthyl)-acétamide, l'alpha-phényl-1H-pyrazole-1-éthanamine, la (+)-N-éthyl-1-phényl-2-(3-pyrazine)-éthanamine ou la 2-amidino-6-(2-amino-2-phényl)-éthylpyridine, ou un de leurs sels pharmaceutiquement acceptables.
